# EUROPEAN PATENT APPLICATION

(11) **EP 4 567 825 A1**
(43) Date of publication of application: **11.06.2025**
(21) Application number: 23383263.3
(22) Date of filing: 05.12.2023
(51) Int. Cl.: G16H 20/70, A61B 5/16, A61M 21/02, A61M 21/00

(54) **SYSTEM AND COMPUTER IMPLEMENTED METHOD FOR IMPROVING A PSYCHOLOGICAL AND/OR PHYSICAL STATE IN A SUBJECT BY PROVIDING MUSIC THERAPY**

(71) Applicant: Fundacion Instituto De Investigacion Sanitaria Fundacion Jimenez Diaz, 28040 Madrid (ES)
(72) Inventor: LÁZARO GARCÍA, Alberto, 28040 Madrid (ES); LAINEZ GONZÁLEZ, Daniel, 28040 Madrid (ES); ALONSO DOMÍNGUEZ, Juan Manuel, 28040 Madrid (ES); LLAMAS SILLERO, Maria Pilar, 28040 Madrid (ES)
(74) Representative: Hoffmann Eitle

(57) **Abstract**

The present invention refers to the medical field. Particularly, the present invention refers to a system for improving a psychological and/or physical state in a subject by providing music therapy, and to a computer implemented method configured to select pieces of music according to the psychological and/or physical state of a subject in order to improve a psychological and/or physical state in a subject.

## Description

### FIELD OF THE INVENTION

The present invention refers to the medical field. Particularly, the present invention refers to a system for improving a psychological and/or physical state in a subject by providing music therapy, and to a computer implemented method configured to select pieces of music according to the psychological and/or physical state of a subject.

### STATE OF THE ART

Cancer treatments are associated to a wide variety of psychological and physical symptoms. Hematopoietic stem cell transplantation (HSCT) is frequently performed during the treatment of hematologic malignancies. However, patients undergoing this process may present with symptoms of toxicity and usually experience a decline in quality of life (QoL) and an increase in depression and psychological distress. Furthermore, patients newly diagnosed with acute myeloid leukemia (AML) may present with a high symptom burden, a significant impact on QoL, anxiety, and depression, regardless of the chemotherapy intensity.

In this context, there is an unmet medical need of finding non-invasive strategies aimed at improving the psychological and/or physical state in a subject. In particular, there is a need of finding non-invasive strategies aimed at improving the psychological and/or physical state in patients undergoing cancer treatment, such as HSCT and intensive induction chemotherapy for AML. The present invention is focused on solving this problem and a non-invasive system for improving a psychological and/or physical state in a subject is herein provided.

### DESCRIPTION OF THE INVENTION

### Brief description of the invention

The present invention refers to a system for improving a psychological and/or physical state in a subject by providing music therapy, and to a computer implemented method configured to select pieces of music according to the psychological and/or physical state of a subject.

The inventors developed a platform to provide music therapy (MT) sessions and evaluated their effect on the psychological and/or physical state of patients undergoing cancer treatment. More precisely, they evaluated the effects of these sessions in patients undergoing HSCT, and patients undergoing intensive induction chemotherapy for AML.

Prior to each MT session, the participant selected one out of three possible groups of emotions depending on how they were feeling that day:
1. Sad, discouraged, or lonely.
2. Nervous, worried, angry, or scared.
3. Bored, fed up, or not wanting to do anything.

A specific MT session was provided based on the selected emotion. The sessions comprises five pieces of classical music ordered by increasing or decreasing beats per minute (BPM) and keys. The specific tempo and key of the pieces selected for each emotion are shown in **Table 1.**

To evaluate the effects of the MT sessions on the psychological and physical effect of the subject, participants were asked to complete questionnaires before and after each MT session.

When considering all the patients regardless of the cancer treatment they were undergoing, MT sessions **(Table 6**):
- Reduced the Edmonton Symptom Assessment System-Revised (ESAS-r) score (p<0.001), which is a score based on the following symptoms: pain, tiredness, nausea, depression, anxiety, drowsiness, lack of appetite, sleep, feeling of well-being and shortness of breath. Boredom was included as an additional symptom to the ESAS-r.
- Reduced the total symptom distress score (p<0.001) and the physical score (p<0.001) ESAS-r subscales.
- Reduced or ameliorated pain (p = 0.085), tiredness (p<0.001), drowsiness (p = 0.056), nausea (p=0.004), well-being (p = 0.095) and boredom (p=0.013).

When considering AML patients alone, MT sessions **(Table 6**):
- Reduced the ESAS-r score (p=0.050).
- Reduced the total symptom distress score (p=0.037) and the physical score (p=0.009) ESAS-r subscales.
- Reduced or ameliorated tiredness (p=0.053) and drowsiness (p=0.026).

When considering patients who received an allogeneic stem cell transplant (alloSCT) alone, MT sessions **(Table 6**):
- Reduced the ESAS-r score (p=0.005).
- Reduced the total symptom distress score (p=0.013), the physical score (p=0.027) and the emotional score (p=0.091) ESAS-r subscales.
- Reduced or ameliorated pain (p=0.010), tiredness (p=0.055), nausea (p<0.001), anxiety (p=0.068), well-being (p=0.014) and boredom (p=0.001).

When considering inpatients who received an autologous stem cell transplant (inpatients autoSCT) alone, MT sessions **(Table 6**):
- Reduced the ESAS-r score (p=0.001).
- Reduced the total symptom distress score (p=0.008), the physical score (p=0.073) and the emotional score (p=0.002) ESAS-r subscales.
- Reduced or ameliorated tiredness (p=0.001), depression (p=0.004), anxiety (p=0.007), sleep (p=0.003), well-being (p=0.006) and boredom (p<0.001).

When considering outpatients who received an autologous stem cell transplant (outpatients autoSCT) alone, MT sessions **(Table 6**):
- Reduced or ameliorated tiredness (p=0.044).

In summary, the above-mentioned results show that MT sessions **(Table 6**):
- Reduce the ESAS-r score, symptom distress (analyzed as the total symptom distress ESAS-r subscale), physical symptoms (analyzed as the physical score ESAS-r subscale), emotional symptoms (analyzed as the emotional score ESAS-r subcale), pain, tiredness, drowsiness, nausea, depression, anxiety and boredom.
- Ameliorate sleep disturbances or difficulty sleeping.
- Improve well-being.

Moreover, in all MT arms, patients who selected the first group of emotions (sad) showed improved depression scores (-0.56; SD = 1.55; p < 0.001); those who chose the second group (nervous) showed ameliorated anxiety (-0.46; 2.04; = 0.002); and those who opted for the third group (bored) showed a decrease in boredom levels (-0.52; 1.04; < 0.001). Significant changes in boredom scores were observed for patients who selected that they were feeling nervous (-0.33; 1.31; = 0.012) and sad (-0.67; 1.59; < 0.001).

Consequently, the first embodiment of the present invention refers to a system (hereinafter referred to as the *"system of the invention"*) for improving a psychological and/or physical state in a subject by providing music therapy, wherein the system comprises a processing unit configured to:
a) Receive data regarding a subject's psychological state selected from three groups of emotions: 1) sadness, discouragement and/or loneliness, 2) nervousness, worry, anger and/or fear, and 3) boredom, weariness and/or apathy, wherein the subject's psychological state is a subject-reported psychological state, or an emotion chosen upon questioning the subject;
b) Process the data received regarding the subject's psychological state to select the music therapy;
c) Provide music therapy according to the subject psychological state received in step a);
and wherein the processing unit is characterized in that it is further configured to:
i) Provide a series of pieces of music with increasing bpm values, wherein the first piece of music is characterized by having less than 60 bpm and the last piece of music is characterized by having more 120 bpm, if the system receives in step a) that the subject is suffering from sadness, discouragement and/or loneliness;
ii) Provide a series of pieces of music with decreasing bpm values, wherein the first piece of music is characterized by having more than 120 bpm and the last piece of music is characterized by having less 60 bpm, if the system receives in step a) that the subject is suffering from nervousness, worry, anger and/or fear; or
iii) Provide a series of pieces of music with increasing bpm values, wherein the first piece of music is characterized by having less than 90 bpm and the last piece of music is characterized by having more 150 bpm, if the system receives in step a) that the subject is suffering from boredom, weariness and/or apathy.

In a preferred embodiment, the improvement of a psychological and/or physical state in a subject by providing music therapy comprises reducing the Edmonton Symptom Assessment System-Revised (ESAS-r) score, reducing symptom distress, reducing the total symptom distress ESAS-r subscale, reducing physical symptoms, reducing the physical score ESAS-r subscale, reducing emotional symptoms, reducing the emotional score ESAS-r subscale, reducing pain, reducing tiredness, reducing drowsiness, reducing nausea, reducing depression, reducing anxiety, ameliorate sleep disturbances, improve well-being, and/or reducing boredom.

In a preferred embodiment, the processing unit is characterized in that it is further configured to:
i) Provide a series of five pieces of music with increasing bpm values, wherein the first piece of music is characterized by having less than 60 bpm, the second piece of music is characterized by having 60-80 bpm, the third piece of music is characterized by having 80-100 bpm, the fourth piece of music is characterized by having 100-120 bpm, and the fifth piece of music is characterized by having more than 120 bpm, if the system receives in step a) that the subject is suffering from sadness, discouragement and/or loneliness;
ii) Provide a series of five pieces of music with decreasing bpm values, wherein the first piece of music is characterized by having more than 120 bpm, the second piece of music is characterized by having 100-120 bpm, the third piece of music is characterized by having 80-100 bpm, the fourth piece of music is characterized by having 60-80 bpm, and the fifth piece of music is characterized by having less 60 bpm, if the system receives in step a) that the subject is suffering from nervousness, worry, anger and/or fear; or
iii) Provide a series of five pieces of music with increasing bpm values, wherein the first piece of music is characterized by having less than 90 bpm, the second piece of music is characterized by having 90-110 bpm, the third piece of music is characterized by having 110-130 bpm, the fourth piece of music is characterized by having 130-150 bpm, and the fifth piece of music is characterized by having more than 150 bpm, if the system receives in step a) that the subject is suffering from boredom, weariness and/or apathy.

In a preferred embodiment, the system of the invention is further characterized in that the last piece is in a major key.

In a preferred embodiment, the processing unit is characterized in that it is further configured to:
i) Provide a series of five pieces of music, wherein the first two pieces of music have a minor key and the last two pieces are in a major key, if the system receives in step a) that the subject is suffering from sadness, discouragement and/or loneliness;
ii) Provide a series of five pieces of music, wherein the last two pieces are in a major key, if the system receives in step a) that the subject is suffering from nervousness, worry, anger and/or fear; or
iii) Provide a series of five pieces of music, wherein the last piece is in a major key, if the system receives in step a) that the subject is suffering from boredom, weariness and/or apathy.

In a preferred embodiment, the subject is characterized in that they have been treated with an anticancer treatment, preferably autologous haematopoietic stem cell transplantation (HSCT), allogeneic HSCT and/or intensive induction chemotherapy for the treatment of acute myeloid leukaemia (AML).

In a preferred embodiment, the subject is a patient suffering from AML, a patient that has received an allogeneic stem cell transplant, a patient who has received an autologous stem cell transplant as an inpatient or a patient who has received an autologous stem cell transplant as an outpatient.

The second embodiment of the present invention refers to a computer implemented method configured to select pieces of music according to the psychological and/or physical state of a subject in order to improve a psychological and/or physical state in a subject, the method comprising the following steps:
i) Receive data regarding a subject's psychological state selected from three groups of emotions: 1) sadness, discouragement and/or loneliness, 2) nervousness, worry, anger and/or fear, and 3) boredom, weariness and/or apathy, wherein the subject's psychological state is a subject-reported psychological state, or an emotion chosen upon questioning the subject;
ii) Process the data received regarding the subject's psychological state to select the music therapy;
iii) Provide music therapy according to the subject psychological state received in step a);
and wherein the method is characterized in that:
i) If the system receives in step a) that the subject is suffering from sadness, discouragement and/or loneliness, the system provides a series of pieces of music with increasing bpm values, wherein the first piece of music is characterized by having less than 60 bpm and the last piece of music is characterized by having more 120 bpm;
ii) If the system receives in step a) that the subject is suffering from nervousness, worry, anger and/or fear, the system provides a series of pieces of music with decreasing bpm values, wherein the first piece of music is characterized by having more than 120 bpm and the last piece of music is characterized by having less 60 bpm; or
iii) If the system receives in step a) that the subject is suffering from boredom, weariness and/or apathy, the system provides a series of pieces of music with increasing bpm values, wherein the first piece of music is characterized by having less than 90 bpm and the last piece of music is characterized by having more 150 bpm.

In a preferred embodiment, the improvement of a psychological and/or physical state in a subject by providing music therapy comprises reducing the ESAS-r score, reducing symptom distress, reducing the total symptom distress ESAS-r subscale, reducing physical symptoms, reducing the physical score ESAS-r subscale, reducing emotional symptoms, reducing the emotional score ESAS-r subscale, reducing pain, reducing tiredness, reducing drowsiness, reducing nausea, reducing depression, reducing anxiety, ameliorate sleep disturbances, improve well-being and/or reducing boredom.

In a preferred embodiment, the computer implemented method of the invention is characterized in that:
i) If the system receives in step a) that the subject is suffering from sadness, discouragement and/or loneliness, the system provides a series of five pieces of music with increasing bpm values, wherein the first piece of music is characterized by having less than 60 bpm, the second piece of music is characterized by having 60-80 bpm, the third piece of music is characterized by having 80-100 bpm, the fourth piece of music is characterized by having 100-120 bpm, and the fifth piece of music is characterized by having more than 120 bpm;
ii) If the system receives in step a) that the subject is suffering from nervousness, worry, anger and/or fear, the system provides a series of five pieces of music with decreasing bpm values, wherein the first piece of music is characterized by having more than 120 bpm, the second piece of music is characterized by having 100-120 bpm, the third piece of music is characterized by having 80-100 bpm, the fourth piece of music is characterized by having 60-80 bpm, and the fifth piece of music is characterized by having less 60 bpm; or
iii) If the system receives in step a) that the subject is suffering from boredom, weariness and/or apathy, the system provides a series of five pieces of music with increasing bpm values, wherein the first piece of music is characterized by having less than 90 bpm, the second piece of music is characterized by having 90-110 bpm, the third piece of music is characterized by having 110-130 bpm, the fourth piece of music is characterized by having 130-150 bpm, and the fifth piece of music is characterized by having more than 150 bpm.

In a preferred embodiment, the computer implemented method of the invention is further characterized in that the last piece is in a major key.

In a preferred embodiment, the computer implemented method of the invention is further characterized in that:
i) If the system receives in step a) that the subject is suffering from sadness, discouragement and/or loneliness the system provides a series of five pieces of music, wherein the first two pieces of music have a minor key and the last two pieces are in a major key;
ii) If the system receives in step a) that the subject is suffering from nervousness, worry, anger and/or fear the system provides a series of five pieces of music, wherein the last two pieces are in a major key; or
iii) If the system receives in step a) that the subject is suffering from boredom, weariness and/or apathy the system provides a series of five pieces of music, wherein the last piece is in a major key.

In a preferred embodiment, the subject is characterized in that they have been treated with an anticancer treatment, preferably autologous HSCT, allogeneic HSCT and/or intensive induction chemotherapy for the treatment of AML.

In a preferred embodiment, the subject is a patient suffering from AML, a patient that has received an allogeneic stem cell transplant, a patient who has received an autologous stem cell transplant as an inpatient or a patient who has received an autologous stem cell transplant as an outpatient.

In a preferred embodiment the music is classical music.

In a preferred embodiment the series of pieces of music have a total duration of 19 to 39 minutes.

The third embodiment of the invention refers to a computer implemented program comprising instructions which, when executed by a processing unit, configures the processing unit to execute the computer implemented method of the invention.

### In the context of the present invention the following terms are defined:

- The term "comprising" means including, but it is not limited to, whatever follows the word "comprising". Thus, use of the term "comprising" indicates that the listed elements are required or mandatory, but that other elements are optional and may or may not be present.
- By "consisting of" means including, and it is limited to, whatever follows the phrase "consisting of". Thus, the phrase "consisting of" indicates that the listed elements are required or mandatory, and that no other elements may be present.
- The term "improving a psychological and/or physical state" refers to modulating the psychological and/or physical state in the direction that has a positive impact on the health of the patent. For instance, "improving a psychological and/or physical state" includes reducing the Edmonton Symptom Assessment System-Revised (ESAS-r) score, reducing symptom distress, reducing the total symptom distress ESAS-r subscale, reducing physical symptoms, reducing the physical score ESAS-r subscale, reducing emotional symptoms, reducing the emotional score ESAS-r subscale, reducing pain, reducing tiredness, reducing drowsiness, reducing nauseas, reducing depression, reducing anxiety, ameliorate sleep disturbances, improve well-being and/or reducing boredom.
- The term "music therapy", as defined by the World Federation of Music Therapy, refers to the professional use of music and its elements as an intervention in medical, educational, and everyday environments with individuals, groups, families, or communities who seek to optimize their quality of life and improve their physical, social, communicative, emotional, intellectual, and spiritual health and wellbeing.
- The term "piece" refers to a musical composition, or a fragment thereof, that has a duration that ranges from 1 to 10 minutes, preferably from 3 to 10 minutes. For example, the shortest piece lasts 3 minutes 35 seconds and the longest piece 9 minutes 47 seconds.

### Description of the figures

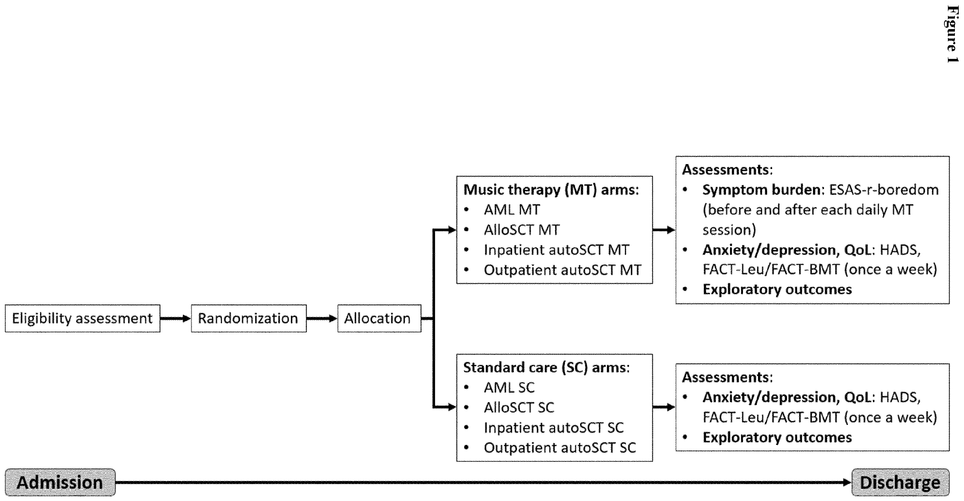

**Figure 1****.** Overview of the study design and participants' assessments. Abbreviations: AML, acute myeloid leukemia; alloSCT, allogeneic stem cell transplantation; autoSCT, autologous stem cell transplantation; MT, music therapy; SC, standard care; ESAS-r, Edmonton Symptom Assessment System-Revised; QoL, quality of life; HADS, Hospital Anxiety and Depression Scale; FACT-Leu, Functional Assessment of Cancer Therapy-Leukemia; FACT-BMT, Functional Assessment of Cancer Therapy-Bone Marrow Transplantation.

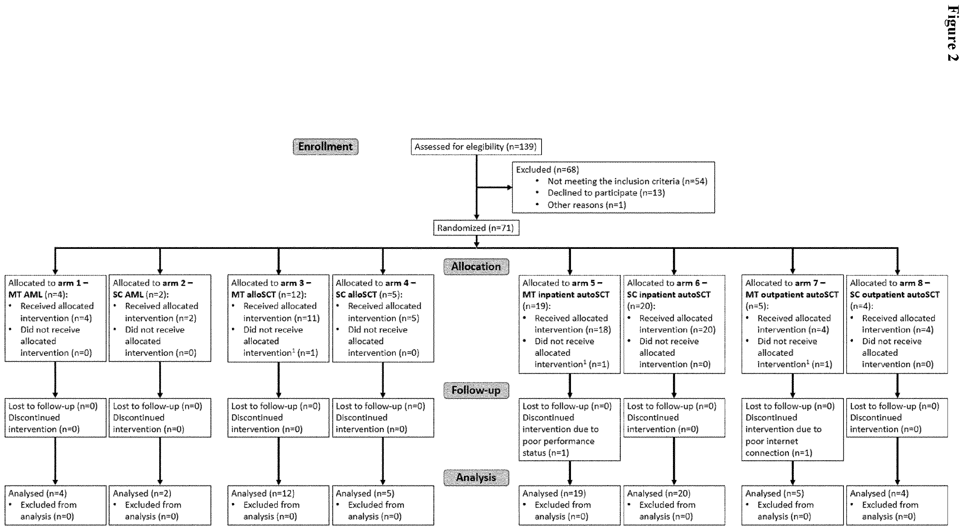

**Figure 2****.** CONSORT flow diagram. ¹ No MT sessions were completed. Abbreviations: MT, music therapy; SC, standard care; AML, acute myeloid leukemia; alloSCT, allogeneic stem cell transplantation; autoSCT, autologous stem cell transplantation.

### Detailed description of the invention

The present invention is illustrated by means of the Examples set below without the intention of limiting its scope of protection.

### Example 1. Material and Methods

### Example 1.1. Study design

The preset study was conducted from February 2021 to November 2022 at the Department of Hematology of Fundación Jiménez Diaz University Hospital, Madrid, Spain.

On the day of admission, patients were invited to participate in the trial. Informed consent was obtained within the first two days. Subsequently, the participants underwent computer-generated randomization and were assigned to either experimental groups (MT with standard supportive care) or control groups (standard supportive care). Randomization was stratified according to the reason for admission (inpatient autoSCT, outpatient autoSCT, alloSCT, or AML) with a 1:1 allocation ratio (**Figure 1**).

A mobile app was designed for this study. Each participant was provided a login ID and password to access the app and answer the study questionnaires. The mobile app was also used to conduct the MT sessions.

### Example 1.2. Participants

The inclusion criteria were: 1) hospitalized patients undergoing HSCT or receiving induction chemotherapy for newly diagnosed AML (excluding acute promyelocytic leukemia); patients included in our outpatient autoSCT program were also eligible; 2) age above 18 years; 3) Spanish language proficiency; and 4) basic computer skills. The exclusion criteria were: 1) moderate or severe hearing impairment (> 40 decibels); 2) previous diagnosis of any psychiatric disorder; 3) impaired performance status at admission (Eastern Cooperative Oncology Group [ECOG] >_ 3); and 4) previous participation in this study.

### Example 1.3. Intervention

### Experimental groups

Each patient included in the experimental groups was provided with a tablet (Alldocube iPlay 8 Pro/iPlay 7T [Guangzhou, Guangdong, China], or SPC Lightyear [Vitoria, Álava, Spain]) with the mobile app already installed and high-quality headphones (Sennheiser HD 400S [Wedemark, Hannover, Germany]).

Upon admission, the participants were invited to undergo daily MT sessions provided by the mobile app. The intervention comprised listening to pre-recorded classical music following a strategy that leads to a change in the mood of patients listening to music. Individuals start listening to music that is "equal" to their initial mood state, and subsequently, continue listening to specific pieces, in which musical elements are gradually changed to achieve the desired positive mood. The music was pre-selected by a member of the research team having an extensive musical background and professional music qualification.

Before the session began, the participants had to select one out of three possible groups of emotions, depending on how they were feeling that day: 1) sad, discouraged, or lonely; 2) nervous, worried, angry, or scared; or 3) bored, fed up, or not wanting to do anything. According to this choice, the participants listened to five pieces (automatically selected by the mobile app), ordered by beats per minute (BPM) and keys, to reach the corresponding positive emotions: 1) energized and joyful; 2) relaxed and calm; and 3) entertained and stimulated **(Table 1**). A general assessment of the pieces according to other musical elements was also conducted to classify them appropriately. To improve the participants' experience, the participants could ask for an alternative piece with similar musical features. However, it could be requested only once during each piece. Once the session was completed, the patients answered a dichotomous question regarding the session satisfaction.

The sessions' median duration for the first group of emotions was 24 min 38 s (minimum: 19 min 37 s; maximum: 34 min 12 s); 24 min 56 s for the second group (18 min 53 s; 38 min 41 s); and 23 min 26 s for the third group (19 min 35 s; 35 min 10 s).

The patients underwent the MT sessions at their preferred time, inside their individual isolation rooms in the hematology ward. The participants controlled the devices' volumes. No specific control was made to limit the decibels.

### Control groups

Participants in the control groups received no musical intervention and underwent standard treatment. These patients could install the mobile app on their devices or were provided with a tablet with the app, so they could complete the study questionnaires.

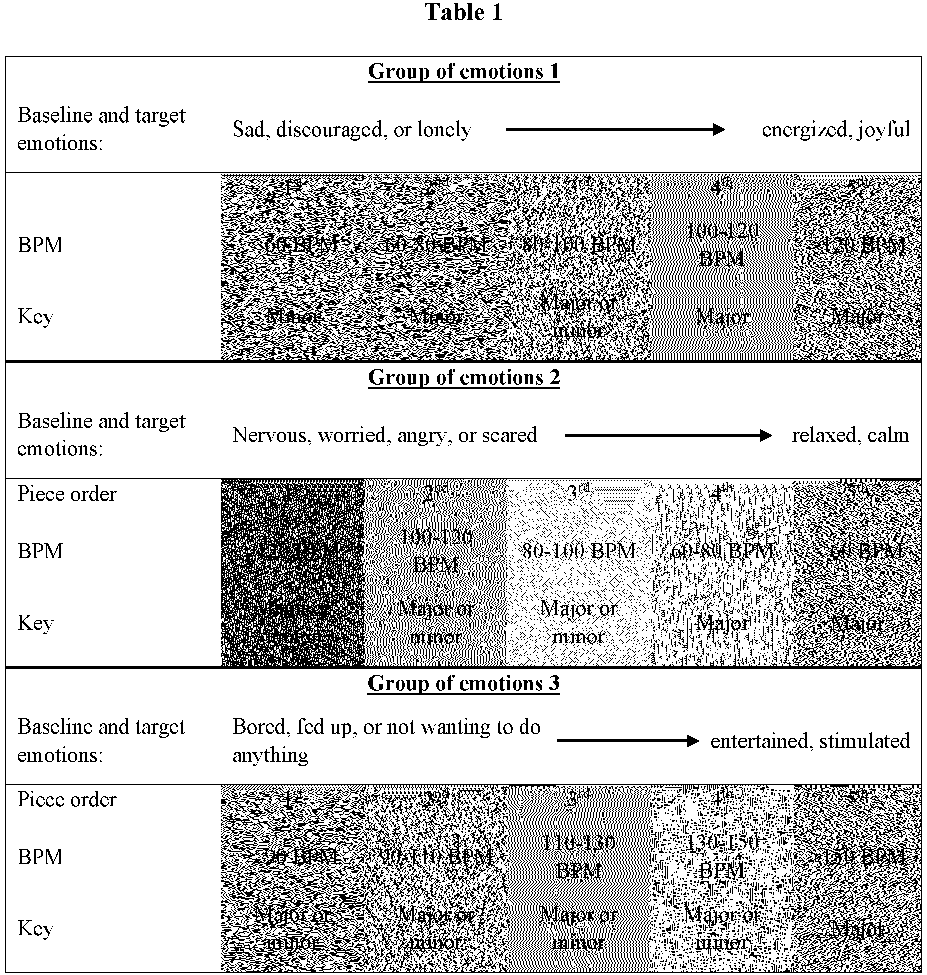

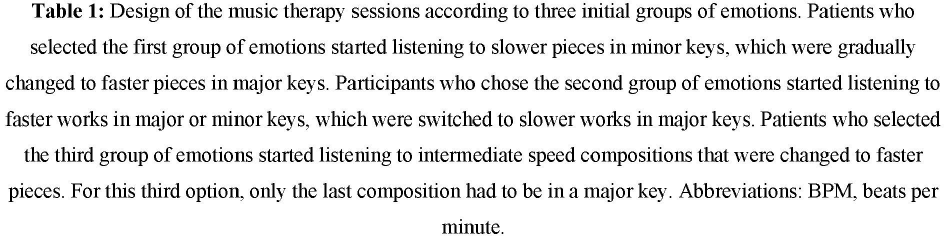

### Example 1.4. Measurement tools

Demographic, musical, clinical, and analytical features, and the use of analgesics, antiemetics, and anxiolytics during admission were recorded.

### Symptom assessment

Participants enrolled in the experimental groups completed the Spanish version of the Edmonton Symptom Assessment System-Revised (ESAS-r), before and after each MT session **(****Figure 1**). Boredom was included as an additional symptom. To prevent the effects of symptom control medication, the mobile app precluded the patients from completing the questionnaire if they declared that they had been administered analgesics, anti-emetics, or anxiolytics within the previous two hours.

The ESAS-r includes ten symptoms on a scale from 0 to 10 (10 = worst possible). The ESAS subscale scores are: physical score (PS; range, 0-60), emotional score (ES; 0-20), and total symptom distress score (TSDS; 0-90). Minimal clinically important differences for improvement were determined for the 10 ESAS-r symptoms (>_ 1) and ESAS subscale scores (PS ≥ 3/60; ES ≥ 2/20; TSDS ≥ 3/90).

### Depression and anxiety

Depression and anxiety were assessed using the Hospital Anxiety and Depression Scale (HADS), whose Spanish version has been validated. HADS includes 14 items, and the total scores range from 0 to 42. High scores imply poor outcomes. Participants included in both experimental and control groups completed the HADS questionnaire once a week until being discharged **(****Figure 1**).

### Quality of life (OoL)

Functional Assessment of Cancer Therapy-Bone Marrow Transplantation (FACT-BMT) was used to assess QoL in HSCT groups, and FACT-Leukemia (FACT-Leu) was used for the leukemia groups. Spanish versions were provided. FACT-BMT includes FACT-General (FACT-G) and a BMT subscale. Similarly, FACT-Leu contains FACT-G and a leukemia subscale. The FACT-G, BMT, and leukemia subscales contain 27, 23, and 17 questions, respectively.

The global QoL score was calculated by adding the FACT-G score (range, 0-108) to the BMT score (0-40) for the HSCT groups or to the leukemia score (0-68) for the leukemia groups. The higher the score, the better was the QoL. Participants included in both experimental and control groups completed FACT-BMT/FACT-Leu once a week until being discharged **(****Figure 1**).

### Example 1.5. Statistical analysis

The inventors calculated that a sample size of 97 MT sessions in the AML, 110 in the alloSCT, 99 in the inpatient autoSCT, and 415 in the outpatient autoSCT experimental groups could enable the detection of an improvement in ESAS-r-boredom scores after the MT sessions (mean difference between ESAS-r-boredom scores post-session and pre-session < 0), with 5% significance (α = 0.05) and 80% power (β = 0.2).

Data were analyzed using descriptive statistics, including mean and standard deviation (SD) or median and interquartile range for continuous variables and frequency and proportion for categorical variables.

Differences between the ESAS-r-boredom, ESAS subscale, and individual symptom scores for the experimental groups at baseline were analyzed using linear mixed models (LMMs). Changes in these scores after MT sessions and differences in the level of anxiety, depression, and boredom depending on the chosen groups of emotions were analyzed using these models.

LMMs were applied to determine differences between HADS, FACT-BMT, and FACT-Leu (and their subscales) scores of the groups (MT vs. standard care [SC]) at baseline and at different weeks of admission. When the interaction effect (group*week) was statistically significant, pairs of groups were compared at each week of admission.

The chi-square test was used to examine the selected emotions, session satisfaction, and proportion of sessions with clinically important differences between the groups. The Chi-square or Fisher's exact test, analysis of variance (ANOVA), independent t-test, Mann-Whitney U test, and Kruskal-Wallis test were used to determine differences in baseline and exploratory variables between the groups.

All data were analyzed on an intention-to-treat basis. Statistical tests were two-sided. Statistical significance was set at p < 0.05. Analysis was performed using SPSS version 28.0 (IBM Corp., Armonk, NY, USA).

### Example 2. Results

### Example 2.1. Participants and baseline measurements

During the recruitment period, 139 patients were assessed for eligibility and 71 were randomized. The patients' reasons for declining to participate were: not wanting to be disturbed during admission (n = 5), preferring to use other sources of entertainment (n = 3), poor performance status (n = 3), disliking music (n = 1), and refusing to use mobile apps (n = 1). Participants were allocated to eight different arms, according to the reason of admission and intervention (**Figure 2**).

Demographic characteristics, musical aspects, and baseline hematological disease (for HSCT recipients) were similar between the arms compared. Many participants were art, music, and classical music enthusiasts. However, few participants played an instrument or had received music education **(Table 2**).

Chemotherapy regimens of all participants in the AML groups comprised idarubicin and cytarabine (3 + 7). Complete response was observed in all patients. Donor types for the SC- and MT-alloSCT groups were human leukocyte antigen (HLA)-identical siblings (20 vs. 25%), haploidentical donors (20 vs. 33.33%), and unrelated donors (60 vs. 41.67%), with no significant differences between groups (p = 0.778). The SC- and MT-alloSCT groups received myeloablative (80 vs. 66.67%) or reduced intensity (20 vs. 33.33%) preparative regimens (p = 1). Participants diagnosed with multiple myeloma received high-dose melphalan as conditioning regimen, whereas patients with lymphoma were administered carmustine, etoposide, cytarabine, and melphalan (BEAM).

The mean ESAS-r-boredom pre-session scores were 38.3 (SD = 11.4), 28.72 (13.76), 37.14 (17.87), and 19.44 (13.55) for the AML, alloSCT, and inpatient and outpatient autoSCT MT groups, respectively, with no significant differences between them (p = 0.424). The ESAS subscale and individual symptom pre-session scores are listed in **Table 3.** The groups had low anxiety and depression and high QoL levels at baseline and similar HADS, FACT-G, FACT-Leu, and FACT-BMT scores **(Table 2; Table 4**).

**Table 2**

| **Variable** | **AML SC** (n=2) | **AML MT** (n=4) | **p** | **AlloSCT SC** (n=5) | **AlloSCT MT** (n=12) | **p** |
|---|---|---|---|---|---|---|
| **Age (years),** mean (SD) | 62.78 (4.63) | 61.22 (8.48) | 0.758^{a} | 46.14 (8.33) | 43.20 (14.16) | 0.563 |
| **Sex,** n (%) | | | 1.000 | | | 0.593 |
| Male | 2 (100.00) | 3 (75.00) | | 3 (60.00) | 4 (33.30) | |
| Female | 0 (0.00) | 1 (25.00) | | 2 (40.00) | 8 (66.70) | |
| **Country of origin,** n (%) | | | . | | | 1.000 |
| Spain | 2 (100.00) | 4 (100.00) | | 5 (100.00) | 11 (91.70) | |
| Other | 0 (0.00) | 0 (0.00) | | 0 (0.00) | 1 (8.30) | |
| **Education,** n (%) | | | 0.333 | | | 0.280 |
| Primary and secondary school | 1 (50.00) | 0 (0.00) | | 2 (40.00) | 9 (75.00) | |
| University | 1 (50.00) | 4 (100.00) | | 3 (60.00) | 3 (25.00) | |
| **Profession,** n (%) ^{b} | | | 0.333 | | | 0.583 |
| Highly qualified | 1 (50.00) | 4 (100.00) | | 2 (40.00) | 2 (16.70) | |
| Medium qualified | 1 (50.00) | 0 (0.00) | | 2 (40.00) | 7 (58.30) | |
| Low qualified | 0 (0.00) | 0 (0.00) | | 1 (20.00) | 3 (25.00) | |
| **Art enthusiast,** n (%) | 1 (50.00) | 3 (75.00) | 1.000 | 5 (100.00) | 10 (83.30) | 1.000 |
| **Music enthusiast,** n (%) | 1 (50.00) | 3 (75.00) | 1.000 | 5 (100.00) | 8 (66.70) | 0.261 |
| **Classical music enthusiast,** n (%) | 1 (50.00) | 1 (25.00) | 1.000 | 3 (60.00) | 3 (25.00) | 0.280 |
| **Music listening (h/week),** mean (SD) | 4.50 (3.54) | 4.25 (3.20) | 0.800^{a} | 8.20 (7.33) | 13.83 (12.28) | 0.506^{a} |
| **Playing an instrument,** n (%) | 0 (0.00) | 0 (0.00) | . | 1 (20.00) | 1 (8.30) | 0.515 |
| **Music education,** n (%) | 0 (0.00) | 0 (0.00) | . | 1 (20.00) | 2 (16.70) | 1.000 |
| **HADS score,** mean (SD)^{c} | 5.00 (n.c.) | 13.00 (5.29) | 0.269 | 6.25 (4.35) | 12.90 (8.31) | 0.161 |
| **FACT-G** score, mean (SD)^{c} | 95.00 (n.c.) | 71.75 (13.84) | 0.230 | 86.75 (10.53) | 77.20 (10.29) | 0.145 |
| **FACT-Leu/FACT-BMT** score, mean (SD) ^{c} | 155.00 (n.c.) | 117.00 (18.42) | 0.162 | 117.25 (13.84) | 103.90 (13.32) | 0.119 |
| **Days of admission,** mean (SD) | 27.00 (4.24) | 31.75 (4.57) | 0.644^{a} | 29.20 (5.89) | 42.92 (10.68) | 0.002 |
| **Hematological disease (stem cell transplantation recipients),** n (%) | | | | | | 0.441 |
| Multiple myeloma | | | | . | . | |
| Non-Hodgkin lymphoma | | | | 1 (20.00) | 2 (16.67) | |
| Hodgkin lymphoma | | | | 0 (0.00) | 1 (8.33) | |
| ALL | | | | 2 (40.00) | 3 (25.00) | |
| AML | | | | 0 (0.00) | 3 (25.00) | |
| MDS | | | | 2 (40.00) | 1 (8.33) | |
| Other | | | | 0 (0.00) | 2 (16.67) | |
| **Age (years),** mean (SD) | 55.04 (8.00) | 56.73 (9.31) | 0.504^{a} | 53.36 (5.56) | 55.81 (4.53) | 0.701 |
| **Sex,** n (%) | | | 0.333 | | | 0.524 |
| Male | 10 (50.00) | 13 (68.40) | | 3 (75.00) | 2 (40.00) | |
| Female | 10 (50.00) | 6(31.60) | | 1 (25.00) | 3 (60.00) | |
| **Country of origin,** n (%) | | | 1.000 | | | 1.000 |
| Spain | 15 (75.00) | 14 (73.70) | | 3 (75.00) | 3 (60.00) | |
| Other | 5 (25.00) | 5 (26.30) | | 1 (25.00) | 2 (40.00) | |
| **Education,** n (%) | | | 0.752 | | | 1.000 |
| Primary and secondary school | 11 (55.00) | 9 (47.40) | | 1 (25.00) | 2 (40.00) | |
| University | 9 (45.00) | 10 (52.60) | | 3 (75.00) | 3 (60.00) | |
| **Profession,** n (%) ^{b} | | | 0.980 | | | 0.347 |
| Highly qualified | 7 (35.00) | 7 (36.80) | | 3 (75.00) | 2 (40.00) | |
| Medium qualified | 8 (40.00) | 7 (36.80) | | 0 (0.00) | 2 (40.00) | |
| Low qualified | 5 (25.00) | 5 (26.30) | | 1 (25.00) | 1 (20.00) | |
| **Art enthusiast,** n (%) | 20 (100.00) | 17 (89.50) | 0.231 | 4 (100.00) | 5 (100.00) | |
| **Music enthusiast,** n (%) | 16 (80.00) | 12 (63.20) | 0.301 | 4 (100.00) | 5 (100.00) | |
| **Classical music enthusiast,** n (%) | 10 (50.00) | 9 (47.40) | 1.000 | 2 (50.00) | 4 (80.00) | 0.524 |
| **Music listening (h/week),** mean (SD) | 15.75 (32.28) | 7.16 (8.30) | 0.120^{a} | 16.75 (14.10) | 18.00 (19.12) | 0.917 |
| **Playing an instrument,** n (%) | 3 (15.00) | 1 (5.30) | 0.605 | 1 (25.00) | 0 (0.00) | 0.444 |
| **Music education,** n (%) | 8 (40.00) | 4 (21.10) | 0.301 | 2 (50.00) | 1 (20.00) | 0.524 |
| **HADS score,** mean (SD) ^{c} | 11.42 (7.14) | 10.83 (6.54) | 0.796 | 7.33 (6.66) | 9.50 (4.66) | 0.631 |
| **FACT-G score,** mean (SD)^{c} | 71.35 (12.70) | 70.94 (11.61) | 0.919 | 80.33 (16.92) | 74.75 (18.48) | 0.699 |
| **FACT-Leu/FACT-BMT score,** mean (SD) ^{c} | 95.45 (16.53) | 94.89 (16.56) | 0.917 | 107.33 (22.19) | 99.50 (26.40) | 0.696 |
| **Days of admission,** mean (SD) | 23.10 (10.66) | 21.00 (3.62) | 0.958^{a} | 20.00 (1.41) | 19.60 (0.55) | 0.879^{a} |
| **Hematological disease (stem cell transplantation recipients),** n (%) | | | 0.695 | | | 0.556 |
| Multiple myeloma | 14 (70.00) | 11 (57.89) | | 4 (100.00) | 4 (80.00) | |
| Non-Hodgkin lymphoma | 4 (20.00) | 5 (26.32) | | 0 (0.00) | 1 (20.00) | |
| Hodgkin lymphoma | 2 (10.00) | 2 (10.53) | | 0 (0.00) | 0 (0.00) | |
| ALL | . | . | | . | . | |
| AML | . | . | | . | . | |
| MDS | . | . | | . | . | |
| Other | 0 (0.00) | 1 (5.26) | | 0 (0.00) | 0(0.00) | |

**Table 2:** Patient's characteristics, and anxiety, depression, and quality of life baseline measurements according to the study arm. Abbreviations: AML, acute myeloid leukemia; alloSCT, allogeneic stem cell transplantation; inpat. autoSCT, inpatient autologous stem cell transplantation; outpat. autoSCT, outpatient autologous stem cell transplantation; MT, music therapy; SC, standard care; HADS, Hospital Anxiety and Depression Scale; FACT-G, Functional Assessment of Cancer Therapy-General; FACT-Leu, Functional Assessment of Cancer Therapy-Leukemia; FACT-BMT, Functional Assessment of Cancer Therapy-Bone Marrow Transplantation; n.c., not calculable; ALL, acute lymphoblastic leukemia; MDS, myelodysplastic syndrome; SD, standard deviation; h, hour. ^{a} Non-parametric tests were performed. ^{b} Highly qualified: directors and intellectual professions; medium qualified: technicians, administrative staff, and commercial workers; low qualified: artisans, machine operators, and elementary occupations. ^{c} Sample size (n) for these variables corresponds to completed baseline questionnaires, specified in **Table 4.**

**Table 3**

| **Variable** | **AML MT** (n=47) ^{a} | **AlloSCT MT** (n=141)^{a} | **Inpat. autoSCT MT** (n=172) ^{a} | **Outpat. autoSCT MT** (n=27) ^{a} | **p** |
|---|---|---|---|---|---|
| **ESAS-r-boredom pre-session score,** mean (SD) | 38.30 (11.40) | 28.72 (13.76) | 37.14 (17.87) | 19.44 (13.55) | 0.424 |
| **ESAS-r pre-session score,** mean (SD) | 33.30 (9.76) | 24.94 (12.14) | 32.94 (17.20) | 17.07 (12.67) | 0.412 |

| **Subscale pre-session scores,** mean (SD) | | | | | |
|---|---|---|---|---|---|
| **Total symptom distress score** | 30.43 (10.52) | 22.57 (10.85) | 29.09 (15.21) | 14.96 (11.17) | 0.372 |
| **Physical score** | 19.04 (5.99) | 14.82 (6.99) | 18.67 (9.50) | 9.30 (8.52) | 0.265 |
| **Emotional score** | 6.72 (4.41) | 4.45 (4.12) | 5.92 (4.76) | 2.67 (2.11) | 0.767 |

| **Individual symptom pre-session scores,** mean (SD) | | | | | |
|---|---|---|---|---|---|
| **Pain** | 1.02 (1.29) | 1.11 (1.78) | 1.49 (1.72) | 0.67 (1.21) | 0.731 |
| **Tiredness** | 4.83 (2.09) | 2.84 (2.01) | 3.96 (2.34) | 2.19 (2.66) | 0.315 |
| **Drowsiness** | 4.15 (2.26) | 2.72 (1.95) | 3.35 (2.48) | 1.89 (2.15) | 0.269 |
| **Nausea** | 1.19 (1.39) | 1.79 (1.94) | 3.02 (3.13) | 1.07 (1.92) | 0.439 |
| **Lack of appetite** | 6.40 (2.22) | 5.84 (2.41) | 5.58 (3.57) | 3.44 (2.06) | 0.540 |
| **Shortness of breath** | 1.45 (1.52) | 0.51 (1.65) | 1.28 (2.67) | 0.04 (0.19) | 0.591 |
| **Depression** | 3.00 (2.44) | 2.21 (2.22) | 3.27 (2.45) | 1.30 (1.46) | 0.696 |
| **Anxiety** | 3.72 (2.12) | 2.24 (2.11) | 2.65 (2.65) | 1.37 (1.04) | 0.734 |
| **Sleep** | 2.87 (2.29) | 2.37 (2.07) | 3.85 (2.99) | 2.11 (1.93) | 0.786 |
| **Well-being** | 4.66 (2.66) | 3.31 (1.64) | 4.49 (2.24) | 3.00 (2.00) | 0.438 |
| **Boredom** | 5.00 (2.61) | 3.78 (2.64) | 4.20 (2.60) | 2.37 (1.31) | 0.723 |

**Table 3:** Pre-session symptom burden in experimental groups. Abbreviations: AML, acute myeloid leukemia; alloSCT, allogeneic stem cell transplantation; inpat. autoSCT, inpatient autologous stem cell transplantation; outpat. autoSCT, outpatient autologous stem cell transplantation; MT, music therapy; ESAS-r, Edmonton Symptom Assessment System-Revised; SD, standard deviation. ^{a} Sample size (n) = completed MT sessions.

**Table 4**

| **Variable** | **AML SC** (n=1) ^{a} | **AML MT** (n=4) ^{a} | **p** | **AlloSCT SC** (n=4) ^{a} | **AlloSCT MT** (n=10) ^{a} | **p** |
|---|---|---|---|---|---|---|
| **Anxiety subscale score,** mean (SD) | 4.00 (n.c.) | 8.25 (2.99) | 0.293 | 4.75 (2.63) | 7.70 (4.40) | 0.240 |
| **Depression subscale score,** mean (SD) | 1.00 (n.c.) | 4.75 (2.36) | 0.251 | 1.50 (1.73) | 5.20 (4.29) | 0.127 |
| **HADS score,** mean (SD) | 5.00 (n.c.) | 13.00 (5.29) | 0.269 | 6.25 (4.35) | 12.90 (8.31) | 0.161 |
| **FACT-G score,** mean (SD) | 95.00 (n.c.) | 71.75 (13.84) | 0.230 | 86.75 (10.53) | 77.20 (10.29) | 0.145 |
| **Leukemia/BMT subscale score,** mean (SD) | 60.00 (n.c.) | 45.25 (5.38) | 0.091 | 30.50 (4.04) | 26.70 (4.00) | 0.135 |
| **FACT-Leu/FACT-BMT score,** mean (SD) | 155.00 (n.c.) | 117.00 (18.42) | 0.162 | 117.25 (13.84) | 103.90 (13.32) | 0.119 |

| **Variable** | **Inpat. autoSCT SC** (n=19) ^{a} | **Inpat. autoSCT MT** (n=18) ^{a} | **p** | **Outpat. autoSCT SC** (n=3) ^{a} | **Outpat. autoSCT MT** (n=4) ^{a} | **p** |
|---|---|---|---|---|---|---|
| **Anxiety subscale score,** mean (SD) | 6.63 (3.08) | 5.44 (3.79) | 0.302 | 4.00 (2.65) | 4.25 (1.71) | 0.884 |
| **Depression subscale score,** mean (SD) | 4.79 (4.63) | 5.39 (3.24) | 0.652 | 3.33 (4.04) | 5.25 (3.10) | 0.506 |
| **HADS score,** mean (SD) | 11.42 (7.14) | 10.83 (6.54) | 0.796 | 7.33 (6.66) | 9.50 (4.66) | 0.631 |
| **FACT-G score,** mean (SD) | 71.35 (12.70) ^{b} | 70.94 (11.61) | 0.919 | 80.33 (16.92) | 74.75 (18.48) | 0.699 |
| **Leukemia/BMT subscale score,** mean (SD) | 24.1 (4.19) ^{b} | 23.94 (5.31) | 0.920 | 27.00 (5.29) | 24.75 (7.93) | 0.691 |
| **FACT-Leu/FACT-BMT score,** mean (SD) | 95.45 (16.53) ^{b} | 94.89 (16.56) | 0.917 | 107.33 (22.19) | 99.50 (26.40) | 0.696 |

**Table 4:** Anxiety, depression, and quality of life baseline measurements by study arm. Abbreviations: AML, acute myeloid leukemia; alloSCT, allogeneic stem cell transplantation; inpat. autoSCT, inpatient autologous stem cell transplantation; outpat. autoSCT, outpatient autologous stem cell transplantation; MT, music therapy; SC, standard care; HADS, Hospital Anxiety and Depression Scale; FACT-G, Functional Assessment of Cancer Therapy-General; FACT-Leu, Functional Assessment of Cancer Therapy-Leukemia; FACT-BMT, Functional Assessment of Cancer Therapy-Bone Marrow Transplantation; SD, standard deviation; n.c., not calculable. ^{a} Sample size (n) = completed baseline questionnaires. ^{b} n = 20.

### Example 2.2. Primary outcome

The mean numbers of completed MT sessions were 11.75 (SD = 12.12), 11.75 (9.95), 9.05 (5.64), and 5.4 (6.23) for the AML, alloSCT, and inpatient and outpatient autoSCT MT groups, respectively (p = 0.455). Changes in ESAS-r-boredom scores after MT sessions were similar regardless of MT group, selected group of emotions, session satisfaction, demographic characteristics, and musical aspects **(Table 5**). The symptom burden was significantly reduced in the alloSCT and inpatient autoSCT MT groups after MT sessions (-3.18, SD = 5.23, p = 0.002; and -3.12, 7.25, < 0.001, respectively). The ESAS-r-boredom score decreased when any of the three groups of emotions was selected, with the third cluster (bored) being the most frequently selected (59.9%). Symptom burden was reduced in patients who were satisfied with the MT sessions (82.7%), were art enthusiasts (84%), and did not play an instrument (95.3%). The ESAS-r-boredom scores significantly reduced for all categories, including sex, country of origin, education, profession, music and classical music enthusiast, and music education **(Table 5**). Changes in ESAS-r, subscale, and individual symptom scores after MT sessions by study arm are listed in **Table 6.**

In all experimental groups, after MT sessions, clinically important improvements (CIIs) were observed in the ESAS subscales (TSDS, 37.8; PS, 28.1; ES, 21.7%) and individual symptoms (pain, 34.7; tiredness, 41.4; drowsiness, 39.4; nausea, 39.8; lack of appetite, 27.3; shortness of breath, 28.1; depression, 34.6; anxiety, 32.9; sleep, 26.2; well-being, 36.7; boredom, 42.2%) **(Table 7**).

The proportion of satisfactory sessions was significantly different for the MT groups (p < 0.001) **(Table 7**). The satisfaction was significantly high for the inpatient and outpatient autoSCT groups (87.8 and 100%, respectively). However, patients with AML were less satisfied than expected (57.40%).

The selected emotions were significantly different for the MT groups (p < 0.001) **(Table 7**). Nervous, worried, angry, or scared was chosen frequently by the AML group (46.80%), whereas the other options were selected less often than expected. Significantly few patients in the alloSCT arm chose that they were feeling nervous (14.90%). In all MT arms, patients who selected the first group of emotions (sad) showed improved depression scores (-0.56; SD = 1.55; p < 0.001); those who chose the second group (nervous) showed ameliorated anxiety (-0.46; 2.04; = 0.002); and those who opted for the third group (bored) showed a decrease in boredom levels (-0.52; 1.04; < 0.001). Significant changes in boredom scores were observed for patients who selected that they were feeling nervous (-0.33; 1.31; = 0.012) and sad (-0.67; 1.59; < 0.001).

**Table 5**

| **Variable** | **Category** | **MT sessions,** n (%) | **Mean** (SD) | **p ^{a}** | **Estimated mean,** (95% CI) | **p ^{b}** |
|---|---|---|---|---|---|---|
| **MT group** | AML | 47 (12.10) | -1.38 (5.92) | 0.832 | -3.84 (-7.97, 0.29) | 0.055 |
| | AlloSCT | 141 (36.40) | -3.18 (5.23) | | -3.55 (-5.91, -1.18) | 0.002 |
| | Inpatient autoSCT | 172 (44.40) | -3.12 (7.25) | | -3.24 (-5.08, -1.41) | <0.001 |
| | Outpatient autoSCT | 27 (7.00) | -0.93 (3.25) | | -1.50 (-5.86, 2.86) | 0.480 |
| | All groups | 387 (100.00) | -2.78 (6.22) | | -3.03 (-4.71, -1.36) | <0.001 |
| **Selected group of emotions** | Sad, discouraged, or lonely | 66 (17.10) | -3.94 (8.92) | 0.698 | -3.74 (-5.51, -1.97) | <0.001 |
| | Nervous, worried, angry, or scared | 89 (23.00) | -2.97 (5.90) | | -3.21 (-4.88, -1.53) | <0.001 |
| | Bored, fed up, or not wanting to do anything | 232 (59.90) | -2.38 (5.34) | | -2.99 (-4.29, -1.70) | <0.001 |
| **Session satisfaction** | No | 67 (17.30) | -0.79 (4.54) | 0.085 | -1.80 (-3.71, 0.11) | 0.065 |
| | Yes | 320 (82.70) | -3.19 (6.45) | | -3.43 (-4.62, -2.24) | <0.001 |
| **Sex** | Male | 215 (55.60) | -2.58 (5.80) | 0.573 | -2.92 (-4.55, -1.29) | <0.001 |
| | Female | 172 (44.40) | -3.03 (6.72) | | -3.60 (-5.50, -1.70) | <0.001 |
| **Country of origin** | Spain | 314 (81.10) | -2.67 (5.62) | 0.725 | -3.32 (-4.62, -2.02) | <0.001 |
| | Other | 73 (18.90) | -3.25 (8.36) | | -2.79 (-5.45, -0.13) | 0.040 |
| **Education** | Primary and secondary school | 190 (49.10) | -2.79 (6.44) | 0.519 | -2.84 (-4.57, -1.10) | <0.001 |
| | University | 197 (50.90) | -2.76 (6.02) | | -3.61 (-5.39, -1.83) | <0.001 |
| **Profession ^{c}** | Highly qualified | 135 (34.90) | -2.11 (5.57) | 0.949 | -3.46 (-5.61, -1.31) | <0.001 |
| | Medium qualified | 176 (45.50) | -3.38 (5.48) | | -3.21 (-5.24, -1.18) | <0.001 |
| | Low qualified | 76 (19.60) | -2.57 (8.49) | | -2.94 (-5.56, -0.32) | 0.021 |
| **Art enthusiast** | No | 62 (16.00) | -1.44 (6.01) | 0.544 | -2.31 (-5.61, 0.99) | 0.140 |
| | Yes | 325 (84.00) | -3.03 (6.24) | | -3.34 (-4.65, -2.03) | <0.001 |
| **Music enthusiast** | No | 126 (32.60) | -2.18 (5.83) | 0.389 | -2.43 (-4.63, -0.24) | 0.021 |
| | Yes | 261 (67.40) | -3.07 (6.39) | | -3.54 (-5.00, -2.08) | <0.001 |
| **Classical music enthusiast** | No | 221 (57.10) | -2.25 (5.49) | 0.133 | -2.44 (-4.00, -0.88) | 0.001 |
| | Yes | 166 (42.90) | -3.48 (7.03) | | -4.26 (-6.13, -2.39) | <0.001 |
| **Playing an instrument** | No | 369 (95.30) | -2.88 (6.28) | 0.411 | -3.30 (-4.55, -2.05) | <0.001 |
| | Yes | 18 (4.70) | -0.61 (4.31) | | -0.61 (-7.36, 6.14) | 0.846 |
| **Music education** | No | 323 (83.50) | -2.68 (6.20) | 0.868 | -3.26 (-4.54, -1.98) | <0.001 |
| | Yes | 64 (16.50) | -3.25 (6.38) | | -2.99 (-5.87, -0.12) | 0.042 |

**Table 5:** Changes in ESAS-r-boredom scores after music therapy sessions. Abbreviations: MT, music therapy; SD, standard deviation; CI, confidence interval; AML, acute myeloid leukemia; alloSCT, allogeneic stem cell transplantation; autoSCT, autologous stem cell transplantation; ESAS-r, Edmonton Symptom Assessment System-Revised. ^{a} Linear mixed model's p value. ^{b} p value for estimated mean = 0. ^{c} Highly qualified: directors and intellectual professions; medium qualified: technicians, administrative staff, and commercial workers; low qualified: artisans, machine operators, and elementary occupations.

**Table 6**

| **Variable** | **MT group** | **Mean** (SD) | **p ^{a}** | **Estimated mean,** (95% CI) | **p** ^{b} |
|---|---|---|---|---|---|
| **ESAS-r** | AML | -1.34 (5.84) | 0.891 | -3.50 (-7.17, 0.17) | 0.050 |
| | AlloSCT | -2.70 (4.88) | | -2.80 (-4.90, -0.70) | 0.005 |
| | Inpatient autoSCT | -2.42 (6.46) | | -2.52 (-4.15, -0.89) | 0.001 |
| | Outpatient autoSCT | -0.81 (3.14) | | -1.58 (-5.46, 2.31) | 0.405 |
| | All groups | -2.28 (5.67) | | -2.60 (-4.09, -1.11) | <0.001 |
| **Subscales** | | | | | |
| **Total symptom distress score** | AML | -1.32 (5.79) | 0.806 | -3.82 (-7.56, -0.09) | 0.037 |
| | AlloSCT | -2.47 (4.42) | | -2.55 (-4.71, -0.40) | 0.013 |
| | Inpatient autoSCT | -2.00 (5.88) | | -2.12 (-3.79, -0.45) | 0.008 |
| | Outpatient autoSCT | -1.07 (2.57) | | -1.56 (-5.49, 2.36) | 0.415 |
| | All groups | -2.02 (5.20) | | -2.51 (-4.03, -1.00) | <0.001 |
| **Physical score** | AML | -1.66 (4.89) | 0.400 | -3.25 (-5.76, -0.74) | 0.009 |
| | AlloSCT | -1.75 (3.56) | | -1.52 (-2.96, -0.08) | 0.027 |
| | Inpatient autoSCT | -0.96 (3.73) | | -0.96 (-2.08, 0.16) | 0.073 |
| | Outpatient autoSCT | -1.07 (1.96) | | -1.54 (-4.19, 1.11) | 0.239 |
| | All groups | -1.34 (3.74) | | -1.82 (-2.84, -0.80) | <0.001 |
| **Emotional score** | AML | 0.40 (1.54) | 0.420 | 0.14 (-1.05, 1.34) | 0.797 |
| | AlloSCT | -0.38 (1.77) | | -0.53 (-1.20, 0.15) | 0.091 |
| | Inpatient autoSCT | -0.69 (2.63) | | -0.77 (-1.30, -0.24) | 0.002 |
| | Outpatient autoSCT | -0.15 (0.72) | | -0.06 (-1.33, 1.22) | 0.925 |
| | All groups | -0.41 (2.16) | | -0.30 (-0.79, 0.18) | 0.185 |
| **Individual symptoms** | | | | | |
| **Pain** | AML | -0.13 (1.50) | 0.529 | -0.18 (-0.66,0.31) | 0.453 |
| | AlloSCT | -0.33 (1.14) | | -0.34 (-0.61, -0.07) | 0.010 |
| | Inpatient autoSCT | -0.14 (1.02) | | -0.10 (-0.32, 0.12) | 0.342 |
| | Outpatient autoSCT | -0.04 (0.34) | | -0.06 (-0.60, 0.49) | 0.829 |
| | All groups | -0.20 (1.10) | | -0.17 (-0.37, 0.03) | 0.085 |
| **Tiredness** | AML | -0.40 (1.17) | 0.726 | -0.61 (-1.26, 0.03) | 0.053 |
| | AlloSCT | -0.40 (1.13) | | -0.34 (-0.70, 0.03) | 0.055 |
| | Inpatient autoSCT | -0.44 (1.18) | | -0.45 (-0.74, -0.16) | 0.001 |
| | Outpatient autoSCT | -0.44 (0.80) | | -0.71 (-1.40, -0.02) | 0.044 |
| | All groups | -0.42 (1.13) | | -0.53 (-0.79, -0.26) | <0.001 |
| **Drowsiness** | AML | -0.55 (1.92) | 0.211 | -1.04 (-1.98, -0.10) | 0.026 |
| | AlloSCT | -0.04 (1.99) | | 0.09 (-0.44, 0.63) | 0.717 |
| | Inpatient autoSCT | -0.28 (1.25) | | -0.28 (-0.69, 0.14) | 0.174 |
| | Outpatient autoSCT | -0.04 (0.81) | | -0.20 (-1.21, 0.81) | 0.685 |
| | All groups | -0.21 (1.63) | | -0.36 (-0.74, 0.03) | 0.056 |
| **Nausea** | AML | -0.09 (1.33) | 0.015^{c} | -0.11 (-0.61,0.39) | 0.636 |
| | AlloSCT | -0.66 (1.44) | | -0.66 (-0.94, -0.38) | <0.001 |
| | Inpatient autoSCT | -0.02 (1.51) | | -0.03 (-0.26, 0.21) | 0.801 |
| | Outpatient autoSCT | -0.37 (0.74) | | -0.39 (-0.97, 0.20) | 0.190 |
| | All groups | -0.29 (1.45) | | -0.30 (-0.51, -0.08) | 0.004 |
| **Lack of appetite** | AML | -0.40 (0.93) | 0.626 | -0.58 (-1.70, 0.53) | 0.281 |
| | AlloSCT | -0.29 (1.11) | | -0.34 (-0.99, 0.31) | 0.267 |
| | Inpatient autoSCT | -0.13 (1.62) | | -0.20 (-0.70, 0.31) | 0.413 |
| | Outpatient autoSCT | -0.19 (1.27) | | 0.38 (-0.78, 1.54) | 0.501 |
| | All groups | -0.22 (1.35) | | -0.18 (-0.64, 0.27) | 0.397 |
| **Shortness of breath** | AML | -0.09 (0.83) | 0.903 | -0.09 (-0.46, 0.29) | 0.660 |
| | AlloSCT | -0.04 (1.34) | | -0.04 (-0.25, 0.18) | 0.753 |
| | Inpatient autoSCT | 0.05 (1.50) | | 0.05 (-0.15, 0.25) | 0.604 |
| | Outpatient autoSCT | 0.00 (0.00) | | 0.00 (-0.50, 0.50) | 1.000 |
| | All groups | 0.00 (1.31) | | -0.02 (-0.19, 0.16) | 0.847 |
| **Depression score** | AML | 0.06 (0.90) | 0.476 | -0.08 (-0.69, 0.53) | 0.777 |
| | AlloSCT | -0.14 (0.76) | | -0.19 (-0.54, 0.15) | 0.232 |
| | Inpatient autoSCT | -0.35 (1.34) | | -0.38 (-0.64, -0.11) | 0.004 |
| | Outpatient autoSCT | -0.07 (0.68) | | 0.09 (-0.56, 0.73) | 0.784 |
| | All groups | -0.21 (1.07) | | -0.14 (-0.39, 0.11) | 0.229 |
| **Anxiety score** | AML | 0.34 (1.01) | 0.155 | 0.32 (-0.23, 0.88) | 0.196 |
| | AlloSCT | -0.24 (1.41) | | -0.26 (-0.57, 0.05) | 0.068 |
| | Inpatient autoSCT | -0.33 (1.81) | | -0.34 (-0.60, -0.09) | 0.007 |
| | Outpatient autoSCT | -0.07 (0.55) | | -0.08 (-0.72, 0.56) | 0.801 |
| | All groups | -0.20 (1.54) | | -0.09 (-0.32, 0.14) | 0.419 |
| **Sleep** | AML | -0.02 (0.92) | 0.356 | -0.04 (-0.67, 0.60) | 0.900 |
| | AlloSCT | -0.23 (1.40) | | -0.24 (-0.60, 0.11) | 0.155 |
| | Inpatient autoSCT | -0.42 (1.78) | | -0.43 (-0.72, -0.13) | 0.003 |
| | Outpatient autoSCT | 0.26 (1.79) | | 0.18 (-0.54, 0.90) | 0.616 |
| | All groups | -0.26 (1.57) | | -0.13 (-0.40, 0.14) | 0.311 |
| **Well-being** | AML | -0.06 (1.07) | 0.503 | -0.14 (-0.71, 0.43) | 0.589 |
| | AlloSCT | -0.33 (1.13) | | -0.36 (-0.68, -0.05) | 0.014 |
| | Inpatient autoSCT | -0.35 (1.45) | | -0.34 (-0.59, -0.08) | 0.006 |
| | Outpatient autoSCT | 0.15 (1.70) | | 0.11 (-0.52, 0.73) | 0.728 |
| | All groups | -0.28 (1.32) | | -0.18 (-0.42, 0.05) | 0.095 |
| **Boredom** | AML | -0.04 (0.98) | 0.263 | -0.27 (-1.16, 0.62) | 0.496 |
| | AlloSCT | -0.49 (1.17) | | -0.76 (-1.28, -0.25) | 0.001 |
| | Inpatient autoSCT | -0.69 (1.33) | | -0.72 (-1.12, -0.32) | <0.001 |
| | Outpatient autoSCT | -0.11 (0.70) | | 0.13 (-0.79, 1.06) | 0.756 |
| | All groups | -0.50 (1.22) | | -0.41 (-0.77, -0.05) | 0.013 |

**Table** 6: Changes in ESAS-r, subscale, and individual symptom scores after MT sessions by study arm. Abbreviations: MT, music therapy; SD, standard deviation; CI, confidence interval; ESAS-r, Edmonton Symptom Assessment System-Revised; AML, acute myeloid leukemia; alloSCT, allogeneic stem cell transplantation; autoSCT, autologous stem cell transplantation. ^{a} Linear mixed model's p value. ^{b} p value for estimated mean = 0. ^{c} Significant differences were found between alloSCT and inpatient autoSCT groups in terms of pre- and post-session nausea scores.

**Table 7**

| **Variable** | **AML** | **AlloSCT** | **Inpatient autoSCT** | **Outpatient autoSCT** | **All groups** | **p** |
|---|---|---|---|---|---|---|
| **ESAS subscales,** n (%) **^{a}** | | | | | | |
| **Total symptom distress score** | 11 (23.40)⁻ | 67 (47.50)⁺ | 63 (36.80) | 5 (18.50)⁻ | 146 (37.80) | 0.003 |
| **Physical score** | 14 (29.80) | 48 (34.50) | 41 (24.00) | 5 (18.50) | 108 (28.10) | 0.134 |
| **Emotional score** | 3 (6.50)⁻ | 27 (22.70) | 43 (27.70)⁺ | 2 (7.70) | 75 (21.70) | 0.005 |

| **ESAS individual symptoms,** n (%) **^{a}** | | | | | | |
|---|---|---|---|---|---|---|
| **Pain** | 6 (20.00) | 29 (46.80) | 33 (32.00) | 2 (28.60) | 70 (34.70) | 0.063 |
| **Tiredness** | 22 (46.80) | 43 (36.10) | 71 (42.00) | 8 (61.50) | 144 (41.40) | 0.248 |
| **Drowsiness** | 18 (46.20) | 46 (39.00) | 59 (39.10) | 4 (28.60) | 127 (39.40) | 0.695 |
| **Nausea** | 6 (17.60)⁻ | 53 (59.60)⁺ | 35 (28.70)⁻ | 7 (77.80)⁺ | 101 (39.80) | <0.001 |
| **Lack of appetite** | 16 (34.00) | 39 (28.30) | 35 (23.80) | 8 (29.60) | 98 (27.30) | 0.548 |
| **Shortness of breath** | 6 (20.00) | 9 (37.50) | 17 (28.80) | 0 (0.00) | 32 (28.10) | 0.488 |
| **Depression** | 7 (15.90)⁻ | 36 (34.60) | 61 (40.40)⁺ | 6(31.60) | 110 (34.60) | 0.028 |
| **Anxiety** | 6 (13.00)⁻ | 43 (39.40) | 51 (36.70) | 4 (18.20) | 104 (32.90) | 0.004 |
| **Sleep** | 6 (18.80) | 36 (31.00) | 39 (26.20) | 2 (10.00) | 83 (26.20) | 0.169 |
| **Well-being** | 12 (26.10) | 56 (42.10) | 62 (37.30) | 6 (23.10) | 136 (36.70) | 0.111 |
| **Boredom** | 13 (28.90)⁻ | 55 (48.70) | 69 (43.90) | 7 (26.90) | 144 (42.20) | 0.048 |
| **Session satisfaction,** n (%) ^{b} | 27 (57.40)⁻ | 115 (81.60) | 151 (87.80)⁺ | 27 (100.00)⁺ | 320 (82.70) | <0.001 |

| **Selected groups of emotions,** n (%) ^{b} | | | | | | |
|---|---|---|---|---|---|---|
| **Sad, discouraged, or lonely** | 3 (6.40)⁻ | 28 (19.90) | 33 (19.20) | 2 (7.40) | 66 (17.10) | <0.001 |
| **Nervous, worried, angry, or scared** | 22 (46.80)⁺ | 21 (14.90)⁻ | 41 (23.80) | 5 (18.50) | 89 (23.00) | |
| **Bored, fed up, or not wanting to do anything** | 22 (46.80)⁻ | 92 (65.20) | 98 (57.00) | 20 (74.10) | 232 (59.90) | |

**Table 7:** Music therapy sessions with clinically important improvements, session satisfaction, and selected groups of emotions. Abbreviations: AML, acute myeloid leukemia; alloSCT, allogeneic stem cell transplantation; autoSCT, autologous stem cell transplantation; ESAS, Edmonton Symptom Assessment System. ^{a} Music therapy sessions, in which patients had a score ≥ 1 for the respective pre-session ESAS subscales/individual symptoms, were included in the analysis. ^{b} All completed music therapy sessions are included in this section. ⁺ Significantly higher than expected (95% confidence). ⁻ Significantly lower than expected (95% confidence).

### Example 2.3. Secondary outcomes

The inventors analyzed the interactions between the groups (SC: standard care and MT) and weeks of admission to find differences in HADS, FACT-Leu, and FACT-BMT scores, and their subscales. Initially, FACT-G, BMT subscale, and FACT-BMT scores of outpatient autoSCT groups were significantly different (**Table 8**)**.** However, no differences were observed when the scores for each week of admission were compared.

**Table 8**

| **Scale /subscale** | **Week of admission** | **AML SC** | **AML MT** | **SC & MT groups** | **p ^{a}** |
|---|---|---|---|---|---|
| **Anxiety subscale** | Week 1, mean (SD) | 4.00 (n.c.) | 8.25 (2.99) | 7.40 (3.21) | n.c. |
| | Week 2, mean (SD) | | 5.00 (2.65) | 5.00 (2.65) | |
| | Week 3, mean (SD) | | 4.50 (3.87) | 4.50 (3.87) | |
| | Week 4, mean (SD) | | 6.75 (3.50) | 6.75 (3.50) | |
| | Week 5, mean (SD) | | 3.00 (0.00) | 3.00 (0.00) | |
| | All weeks, mean (SD) | | 5.67 (3.27) | 5.58 (3.20) | |
| **Depression subscale** | Week 1, mean (SD) | 1.00 (n.c.) | 4.75 (2.36) | 4.00 (2.65) | n.c. |
| | Week 2, mean (SD) | | 6.67 (5.03) | 6.67 (5.03) | |
| | Week 3, mean (SD) | | 6.50 (4.66) | 6.50 (4.66) | |
| | Week 4, mean (SD) | | 7.25 (2.22) | 7.25 (2.22) | |
| | Week 5, mean (SD) | | 5.67 (3.22) | 5.67 (3.22) | |
| | All weeks, mean (SD) | | 6.17 (3.28) | 5.89 (3.40) | |
| **HADS** | Week 1, mean (SD) | 5.00 (n.c.) | 13.00 (5.29) | 11.40 (5.81) | n.c. |
| | Week 2, mean (SD) | | 11.67 (7.64) | 11.67 (7.64) | |
| | Week 3, mean (SD) | | 11.00 (6.68) | 11.00 (6.68) | |
| | Week 4, mean (SD) | | 14.00 (2.16) | 14.00 (2.16) | |
| | Week 5, mean (SD) | | 8.67 (3.22) | 8.67 (3.22) | |
| | All weeks, mean (SD) | | 11.83 (5.01) | 11.47 (5.11) | |
| **FACT-G** | Week 1, mean (SD) | 95.00 (n.c.) | 71.75 (13.84) | 76.40 (15.87) | n.c. |
| | Week 2, mean (SD) | | 65.33 (12.06) | 65.33 (12.06) | |
| | Week 3, mean (SD) | | 65.25 (14.80) | 65.25 (14.80) | |
| | Week 4, mean (SD) | | 62.25 (2.99) | 62.25 (2.99) | |
| | Week 5, mean (SD) | | 75.67 (4.73) | 75.67 (4.73) | |
| | All weeks, mean (SD) | | 67.78 (10.87) | 69.21 (12.27) | |
| **Leukemia subscale** | Week 1, mean (SD) | 60.00 (n.c.) | 45.25 (5.38) | 48.20 (8.08) | n.c. |
| | Week 2, mean (SD) | | 37.67 (11.93) | 37.67 (11.93) | |
| | Week 3, mean (SD) | | 42.25 (7.63) | 42.25 (7.63) | |
| | Week 4, mean (SD) | | 39.00 (6.22) | 39.00 (6.22) | |
| | Week 5, mean (SD) | | 48.00 (5.00) | 48.00 (5.00) | |
| | All weeks, mean (SD) | | 42.39 (7.48) | 43.32 (8.32) | |
| **FACT-Leu** | Week 1, mean (SD) | 155.00 (n.c.) | 117.00 (18.42) | 124.60 (23.31) | n.c. |
| | Week 2, mean (SD) | | 103.00 (23.90) | 103.00 (23.90) | |
| | Week 3, mean (SD) | | 107.50 (22.40) | 107.50 (22.40) | |
| | Week 4, mean (SD) | | 101.25 (8.88) | 101.25 (8.88) | |
| | Week 5, mean (SD) | | 123.67 (9.61) | 123.67 (9.61) | |
| | All weeks, mean (SD) | | 110.17 (17.69) | 112.53 (20.03) | |

| **Scale /subscale** | **Week of admission** | **AlloSCT SC** | **AlloSCT MT** | **SC & MT groups** | **p ^{a}** |
|---|---|---|---|---|---|
| **Anxiety subscale** | Week 1, mean (SD) | 4.75 (2.63) | 7.70 (4.40) | 6.86 (4.11) | 0.512 |
| | Week 2, mean (SD) | 3.80 (2.59) | 7.33 (2.96) | 6.07 (3.25) | |
| | Week 3, mean (SD) | 3.50 (3.11) | 6.40 (3.63) | 5.57 (3.63) | |
| | Week 4, mean (SD) | 4.00 (3.37) | 5.90 (3.11) | 5.36 (3.18) | |
| | Week 5, mean (SD) | 8.00 (6.56) | 7.25 (4.74) | 7.45 (4.95) | |
| | Week 6, mean (SD) | 1.00 (n.c.) | 5.80 (4.55) | 5.00 (4.52) | |
| | All weeks, mean (SD) | 4.43 (3.56) | 6.75 (3.72) | 6.09 (3.80) | |
| **Depression subscale** | Week 1, mean (SD) | 1.50 (1.73) | 5.20 (4.29) | 4.14 (4.06) | 0.501 |
| | Week 2, mean (SD) | 5.00 (2.55) | 6.00 (3.94) | 5.64 (3.43) | |
| | Week 3, mean (SD) | 4.75 (1.71) | 7.20 (4.26) | 6.50 (3.82) | |
| | Week 4, mean (SD) | 6.25 (3.78) | 8.20 (4.24) | 7.64 (4.07) | |
| | Week 5, mean (SD) | 11.00 (6.25) | 9.50 (5.32) | 9.91 (5.30) | |
| | Week 6, mean (SD) | 3.00 (n.c.) | 8.20 (6.10) | 7.33 (5.85) | |
| | All weeks, mean (SD) | 5.29 (4.06) | 7.32 (4.57) | 6.74 (4.50) | |
| **HADS** | Week 1, mean (SD) | 6.25 (4.35) | 12.90 (8.31) | 11.00 (7.86) | 0.493 |
| | Week 2, mean (SD) | 8.80 (3.63) | 13.33 (6.80) | 11.71 (6.13) | |
| | Week 3, mean (SD) | 8.25 (4.57) | 13.60 (7.28) | 12.07 (6.91) | |
| | Week 4, mean (SD) | 10.25 (6.95) | 14.10 (6.47) | 13.00 (6.59) | |
| | Week 5, mean (SD) | 19.00 (12.53) | 16.75 (9.97) | 17.36 (10.10) | |
| | Week 6, mean (SD) | 4.00 (n.c.) | 14.00 (10.42) | 12.33 (10.17) | |
| | All weeks, mean (SD) | 9.71 (7.02) | 14.08 (7.67) | 12.84 (7.70) | |
| **FACT-G** | Week 1, mean (SD) | 86.75 (10.53) | 77.20 (10.29) | 79.93 (10.91) | 0.565 |
| | Week 2, mean (SD) | 70.20 (6.94) | 73.78 (9.54) | 72.50 (8.60) | |
| | Week 3, mean (SD) | 69.75 (6.85) | 64.00 (10.55) | 65.64 (9.76) | |
| | Week 4, mean (SD) | 66.75 (11.44) | 61.30 (11.72) | 62.86 (11.48) | |
| | Week 5, mean (SD) | 68.00 (14.14) | 62.62 (15.65) | 63.70 (14.76) | |
| | Week 6, mean (SD) | 81.00 (n.c.) | 57.00 (11.51) | 61.00 (14.21) | |
| | All weeks, mean (SD) | 73.05 (11.16) | 66.83 (12.92) | 68.53 (12.70) | |
| **BMT subscale** | Week 1, mean (SD) | 30.50 (4.04) | 26.70 (4.00) | 27.79 (4.25) | 0.894 |
| | Week 2, mean (SD) | 24.00 (3.08) | 22.44 (3.75) | 23.00 (3.49) | |
| | Week 3, mean (SD) | 25.25 (1.89) | 20.90 (4.33) | 22.14 (4.24) | |
| | Week 4, mean (SD) | 24.25 (5.56) | 19.30 (4.17) | 20.71 (4.95) | |
| | Week 5, mean (SD) | 23.50 (3.54) | 19.63 (6.32) | 20.40 (5.93) | |
| | Week 6, mean (SD) | 27.00 (n.c.) | 19.60 (3.21) | 20.83 (4.17) | |
| | All weeks, mean (SD) | 25.70 (4.17) | 21.55 (5.02) | 22.68 (5.12) | |
| **FACT-BMT** | Week 1, mean (SD) | 117.25 (13.84) | 103.90 (13.32) | 107.71 (14.36) | 0.673 |
| | Week 2, mean (SD) | 94.20 (8.90) | 96.22 (12.33) | 95.50 (10.90) | |
| | Week 3, mean (SD) | 95.00 (7.79) | 84.90 (13.89) | 87.79 (13.04) | |
| | Week 4, mean (SD) | 91.00 (16.79) | 80.60 (15.40) | 83.57 (15.91) | |
| | Week 5, mean (SD) | 91.50 (17.68) | 82.25 (21.55) | 84.10 (20.28) | |
| | Week 6, mean (SD) | 108.00 (n.c.) | 76.60 (13.59) | 81.83 (17.67) | |
| | All weeks, mean (SD) | 98.75 (14.85) | 88.38 (17.20) | 91.22 (17.14) | |

| **Scale /subscale** | **Week of admission** | **Inpat. autoSCT SC** | **Inpat. autoSCT MT** | **SC & MT groups** | **p ^{a}** |
|---|---|---|---|---|---|
| **Anxiety subscale** | Week 1, mean (SD) | 6.63 (3.08) | 5.44 (3.79) | 6.05 (3.45) | 0.521 |
| | Week 2, mean (SD) | 6.47 (3.88) | 4.94 (3.80) | 5.73 (3.86) | |
| | Week 3, mean (SD) | 4.81 (2.83) | 4.50 (2.71) | 4.67 (2.73) | |
| | Week 4, mean (SD) | 6.60 (2.70) | 3.17 (2.64) | 4.73 (3.10) | |
| | All weeks, mean (SD) | 6.10 (3.26) | 4.80 (3.42) | 5.47 (3.39) | |
| **Depression subscale** | Week 1, mean (SD) | 4.79 (4.63) | 5.39 (3.24) | 5.08 (3.97) | 0.592 |
| | Week 2, mean (SD) | 5.68 (3.38) | 7.94 (4.29) | 6.78 (3.97) | |
| | Week 3, mean (SD) | 5.81 (3.97) | 7.07 (3.65) | 6.40 (3.81) | |
| | Week 4, mean (SD) | 6.60 (4.39) | 6.00 (4.43) | 6.27 (4.20) | |
| | All weeks, mean (SD) | 5.58 (4.00) | 6.70 (3.88) | 6.12 (3.96) | |
| **HADS** | Week 1, mean (SD) | 11.42 (7.14) | 10.83 (6.54) | 11.14 (6.76) | 0.706 |
| | Week 2, mean (SD) | 12.16 (6.53) | 12.89 (7.44) | 12.51 (6.90) | |
| | Week 3, mean (SD) | 10.63 (5.69) | 11.57 (6.00) | 11.07 (5.75) | |
| | Week 4, mean (SD) | 13.20 (5.63) | 9.17 (7.00) | 11.00 (6.45) | |
| | All weeks, mean (SD) | 11.68 (6.33) | 11.50 (6.68) | 11.59 (6.47) | |
| **FACT-G** | Week 1, mean (SD) | 71.35 (12.70) | 70.94 (11.61) | 71.16 (12.04) | 0.611 |
| | Week 2, mean (SD) | 64.89 (12.84) | 62.17 (14.53) | 63.57 (13.57) | |
| | Week 3, mean (SD) | 67.53 (12.89) | 65.64 (13.56) | 66.68 (13.01) | |
| | Week 4, mean (SD) | 60.40 (14.69) | 68.33 (13.41) | 64.73 (13.91) | |
| | All weeks, mean (SD) | 67.18 (13.05) | 66.52 (13.41) | 66.86 (13.17) | |
| **BMT subscale** | Week 1, mean (SD) | 24.10 (4.19) | 23.94 (5.31) | 24.03 (4.69) | 0.939 |
| | Week 2, mean (SD) | 21.79 (4.87) | 21.33 (5.29) | 21.57 (5.01) | |
| | Week 3, mean (SD) | 21.65 (5.09) | 22.21 (4.44) | 21.90 (4.74) | |
| | Week 4, mean (SD) | 21.20 (3.70) | 21.67 (1.86) | 21.45 (2.70) | |
| | All weeks, mean (SD) | 22.47 (4.64) | 22.43 (4.85) | 22.45 (4.72) | |
| **FACT-BMT** | Week 1, mean (SD) | 95.45 (16.53) | 94.89 (16.56) | 95.18 (16.32) | 0.764 |
| | Week 2, mean (SD) | 86.68 (16.90) | 83.50 (19.30) | 85.14 (17.92) | |
| | Week 3, mean (SD) | 89.18 (17.27) | 87.86 (17.36) | 88.58 (17.03) | |
| | Week 4, mean (SD) | 81.60 (17.21) | 90.00 (15.10) | 86.18 (15.87) | |
| | All weeks, mean (SD) | 89.65 (16.98) | 88.95 (17.71) | 89.31 (17.26) | |

| **Scale /subscale** | **Week of admission** | **Outpat. autoSCT SC** | **Outpat. autoSCT MT** | **SC & MT groups** | **p ^{a}** |
|---|---|---|---|---|---|
| **Anxiety subscale** | Week 1, mean (SD) | 4.00 (2.65) | 4.25 (1.71) | 4.14 (1.95) | 0.596 |
| | Week 2, mean (SD) | 3.25 (2.87) | 4.50 (2.12) | 3.67 (2.50) | |
| | Week 3, mean (SD) | 3.67 (2.08) | 3.00 (1.73) | 3.33 (1.75) | |
| | All weeks, mean (SD) | 3.60 (2.32) | 3.89 (1.69) | 3.74 (2.00) | |
| **Depression subscale** | Week 1, mean (SD) | 3.33 (4.04) | 5.25 (3.10) | 4.43 (3.36) | 0.984 |
| | Week 2, mean (SD) | 4.25 (3.30) | 5.50 (4.95) | 4.67 (3.45) | |
| | Week 3, mean (SD) | 4.00 (5.29) | 5.00 (4.36) | 4.50 (4.37) | |
| | All weeks, mean (SD) | 3.90 (3.70) | 5.22 (3.38) | 4.53 (3.52) | |
| **HADS** | Week 1, mean (SD) | 7.33 (6.66) | 9.50 (4.66) | 8.57 (5.19) | 0.908 |
| | Week 2, mean (SD) | 7.50 (5.69) | 10.00 (7.07) | 8.33 (5.57) | |
| | Week 3, mean (SD) | 7.67 (7.23) | 8.00 (6.08) | 7.83 (5.98) | |
| | All weeks, mean (SD) | 7.50 (5.68) | 9.11 (4.94) | 8.26 (5.26) | |
| **FACT-G** | Week 1, mean (SD) | 80.33 (16.92) | 74.75 (18.48) | 77.14 (16.59) | 0.003 |
| | Week 2, mean (SD) | 77.75 (11.33) | 69.00 (19.80) | 74.83 (13.26) | |
| | Week 3, mean (SD) | 83.00 (12.73) | 64.00 (15.56) | 76.67 (15.55) | |
| | All weeks, mean (SD) | 80.36 (12.24) | 70.63 (16.12) | 76.26 (14.45) | |
| **BMT subscale** | Week 1, mean (SD) | 27.00 (5.29) | 24.75 (7.93) | 25.71 (6.50) | 0.018 |
| | Week 2, mean (SD) | 27.50 (6.61) | 22.50 (2.12) | 25.83 (5.81) | |
| | Week 3, mean (SD) | 29.50 (4.66) | 24.50 (3.54) | 27.83 (4.71) | |
| | All weeks, mean (SD) | 28.09 (5.15) | 24.13 (5.52) | 26.42 (5.53) | |
| **FACT-BMT** | Week 1, mean (SD) | 107.33 (22.19) | 99.50 (26.40) | 102.86 (23.03) | 0.002 |
| | Week 2, mean (SD) | 105.25 (17.69) | 91.50 (21.92) | 100.67 (18.28) | |
| | Week 3, mean (SD) | 112.50 (17.33) | 88.50 (19.09) | 104.50 (20.17) | |
| | All weeks, mean (SD) | 108.45 (17.13) | 94.75 (21.13) | 102.68 (19.62) | |

**Table 8:** HADS, FACT-Leu, FACT-BMT, and subscale scores by week of admission and study arm. Abbreviations: AML, acute myeloid leukemia; alloSCT, allogeneic stem cell transplantation; inpat. autoSCT, inpatient autologous stem cell transplantation; outpat. autoSCT, outpatient autologous stem cell transplantation; MT, music therapy; SC, standard care; HADS, Hospital Anxiety and Depression Scale; FACT-G, Functional Assessment of Cancer Therapy-General; FACT-Leu, Functional Assessment of Cancer Therapy-Leukemia; FACT-BMT, Functional Assessment of Cancer Therapy-Bone Marrow Transplantation; SD, standard deviation; n.c., not calculable. ^{a} Interaction group*week of admission.

### Example 2.4. Exploratory outcomes

The frequency of mucositis, febrile neutropenia, infections (bacterial, fungal, and viral), necessity of total parenteral nutrition (TPN), transfusion requirement, days of admission, ICU admission, and deaths were analyzed. The mean days of admission were higher in the alloSCT MT group compared to that in the SC group (**Table 2**)**.** However, fewer patients in the inpatient autoSCT MT group required TPN (78.9%) compared to those in the SC group (100%) (p = 0.047). No other significant results were observed.

No differences between the SC and MT arms were found regarding the administration and daily dosage of analgesics, antiemetics, and filgrastim. In terms of transplant-specific complications (graft failure, engraftment syndrome, acute graft-versus-host disease, sinusoidal obstruction syndrome, hemorrhagic cystitis, and cytomegalovirus reactivation), no differences were detected between the SC and MT HSCT groups.

Some potential confusion factors were also examined. Whether participants had listened to their own music and for how many hours and if they had practiced other non-pharmacological therapies, including physical exercise, were noted on the day of discharge. No significant differences were detected among the groups **(Table 9**).

**Table 9**

| **Variable** | **AML SC** (n=1) ^{a} | **AML MT** (n=4) ^{a} | **p** | **AlloSCT SC** (n=4) ^{a} | **AlloSCT MT** (n=10) ^{a} | **p** |
|---|---|---|---|---|---|---|
| **Listening to own music,** n (%) | 1 (100.00) | 3 (75.00) | 1.000 | 4 (100.00) | 5 (50.00) | 0.221 |
| **Listening to own music (h/week),** mean (SD) | 25.00 (n.c.) | 10.75 (8.69) | 0.400^{b} | 9.75 (8.02) | 3.30 (5.19) | 0.054^{b} |
| **Use of non-pharmacological therapies,** n (%) | 1 (100.00) | 2 (50.00) | 1.000 | 4 (100.00) | 6 (60.00) | 0.251 |
| **Physical exercise,** n (%) | 1 (100.00) | 2 (50.00) | 1.000 | 4 (100.00) | 3 (30.00) | 0.070 |

| **Variable** | **Inpat. autoSCT SC** (n=19) ^{a} | **Inpat. autoSCT MT** (n=17)^{a} | **p** | **Outpat. autoSCT SC** (n=4) ^{a} | **Outpat. autoSCT MT** (n=2) ^{a} | **p** |
|---|---|---|---|---|---|---|
| **Listening to own music,** n (%) | 13 (68.40) | 13 (76.50) | 0.717 | 3 (75.00) | 1 (50.00) | 1.000 |
| **Listening to own music (h/week),** mean (SD) | 3.00 (4.00) | 16.35 (57.69) | 0.827^{b} | 7.00 (8.04) | 7.00 (9.90) | 0.800^{b} |
| **Use of non-pharmacological therapies,** n (%) | 9 (47.40) | 8 (47.10) | 1.000 | 2 (50.00) | 1 (50.00) | 1.000 |
| **Physical exercise,** n (%) | 7 (36.80) | 5 (29.40) | 0.732 | 1 (25.00) | 1 (50.00) | 1.000 |

**Table 9:** Potential confusion factors. Abbreviations: AML, acute myeloid leukemia; alloSCT, allogeneic stem cell transplantation; inpat. autoSCT, inpatient autologous stem cell transplantation; outpat. autoSCT, outpatient autologous stem cell transplantation; MT, music therapy; SC, standard care; n.c., not calculable; SD, standard deviation; h, hour. ^{a} Sample size (n) = participants with available data at discharge. ^{b} Non-parametric tests were performed.

## Claims

1. A system for improving a psychological and/or physical state in a subject by providing music therapy, wherein the system comprises a processing unit configured to:
i) Receive data regarding a subject's psychological state selected from three groups of emotions: 1) sadness, discouragement and/or loneliness, 2) nervousness, worry, anger and/or fear, and 3) boredom, weariness and/or apathy, wherein the subject's psychological state is a subject-reported psychological state, or an emotion chosen upon questioning the subject;
ii) Process the data received regarding the subject's psychological state to select the music therapy;
iii) Provide music therapy according to the subject psychological state received in step a);
and wherein the processing unit is **characterized in that** it is further configured to:
i) Provide a series of pieces of music with increasing bpm values, wherein the first piece of music is **characterized by** having less than 60 bpm and the last piece of music is **characterized by** having more 120 bpm, if the system receives in step a) that the subject is suffering from sadness, discouragement and/or loneliness;
ii) Provide a series of pieces of music with decreasing bpm values, wherein the first piece of music is **characterized by** having more than 120 bpm and the last piece of music is **characterized by** having less 60 bpm, if the system receives in step a) that the subject is suffering from nervousness, worry, anger and/or fear; or
iii) Provide a series of pieces of music with increasing bpm values, wherein the first piece of music is **characterized by** having less than 90 bpm and the last piece of music is **characterized by** having more 150 bpm, if the system receives in step a) that the subject is suffering from boredom, weariness and/or apathy.

2. System, according to claim 1, wherein the improvement of a psychological and/or physical state in a subject by providing music therapy comprises reducing the Edmonton Symptom Assessment System-Revised (ESAS-r) score, reducing symptom distress, reducing the total symptom distress ESAS-r subscale, reducing physical symptoms, reducing the physical score ESAS-r subscale, reducing emotional symptoms, reducing the emotional score ESAS-r subscale, reducing pain, reducing tiredness, reducing drowsiness, reducing nausea, reducing depression, reducing anxiety, ameliorate sleep disturbances, improve well-being and/or reducing boredom.

3. System, according to any of the previous claims, wherein processing unit is **characterized in that** it is further configured to:
i) Provide a series of five pieces of music with increasing bpm values, wherein the first piece of music is **characterized by** having less than 60 bpm, the second piece of music is **characterized by** having 60-80 bpm, the third piece of music is **characterized by** having 80-100 bpm, the fourth piece of music is **characterized by** having 100-120 bpm, and the fifth piece of music is **characterized by** having more than 120 bpm, if the system receives in step a) that the subject is suffering from sadness, discouragement and/or loneliness;
ii) Provide a series of five pieces of music with decreasing bpm values, wherein the first piece of music is **characterized by** having more than 120 bpm, the second piece of music is **characterized by** having 100-120 bpm, the third piece of music is **characterized by** having 80-100 bpm, the fourth piece of music is **characterized by** having 60-80 bpm, and the fifth piece of music is **characterized by** having less 60 bpm, if the system receives in step a) that the subject is suffering from nervousness, worry, anger and/or fear; or
iii) Provide a series of five pieces of music with increasing bpm values, wherein the first piece of music is **characterized by** having less than 90 bpm, the second piece of music is **characterized by** having 90-110 bpm, the third piece of music is **characterized by** having 110-130 bpm, the fourth piece of music is **characterized by** having 130-150 bpm, and the fifth piece of music is **characterized by** having more than 150 bpm, if the system receives in step a) that the subject is suffering from boredom, weariness and/or apathy.

4. System, according to any of the previous claims, further **characterized in that** the last piece is in a major key.

5. System, according to any of the previous claims, wherein processing unit is **characterized in that** it is further configured to:
i) Provide a series of five pieces of music, wherein the first two pieces of music have a minor key and the last two pieces are in a major key, if the system receives in step a) that the subject is suffering from sadness, discouragement and/or loneliness;
ii) Provide a series of five pieces of music, wherein the last two pieces are in a major key, if the system receives in step a) that the subject is suffering from nervousness, worry, anger and/or fear; or
iii) Provide a series of five pieces of music, wherein the last piece is in a major key, if the system receives in step a) that the subject is suffering from boredom, weariness and/or apathy.

6. System, according to any of the previous claims, wherein the subject is **characterized in that** they have been treated with an anticancer treatment, preferably autologous haematopoietic stem cell transplantation (HSCT), allogeneic HSCT and/or intensive induction chemotherapy for the treatment of acute myeloid leukaemia (AML).

7. System, according to any of the previous claims, wherein the subject is a patient suffering from AML, a patient that has received an allogeneic stem cell transplant, a patient who has received an autologous stem cell transplant as an inpatient, or a patient who has received an autologous stem cell transplant as an outpatient.

8. A computer implemented method configured to select pieces of music according to the psychological and/or physical state of a subject in order to improve a psychological and/or physical state in a subject, the method comprising the following steps:
i) Receive data regarding a subject's psychological state selected from three groups of emotions: 1) sadness, discouragement and/or loneliness, 2) nervousness, worry, anger and/or fear, and 3) boredom, weariness and/or apathy, wherein the subject's psychological state is a subject-reported psychological state, or an emotion chosen upon questioning the subject;
ii) Process the data received regarding the subject's psychological state to select the music therapy;
iii) Provide music therapy according to the subject psychological state received in step a);
and wherein the method is **characterized in that**:
i) If the system receives in step a) that the subject is suffering from sadness, discouragement and/or loneliness, the system provides a series of pieces of music with increasing bpm values, wherein the first piece of music is **characterized by** having less than 60 bpm and the last piece of music is **characterized by** having more 120 bpm;
ii) If the system receives in step a) that the subject is suffering from nervousness, worry, anger and/or fear, the system provides a series of pieces of music with decreasing bpm values, wherein the first piece of music is **characterized by** having more than 120 bpm and the last piece of music is **characterized by** having less 60 bpm; or
iii) If the system receives in step a) that the subject is suffering from boredom, weariness and/or apathy, the system provides a series of pieces of music with increasing bpm values, wherein the first piece of music is **characterized by** having less than 90 bpm and the last piece of music is **characterized by** having more 150 bpm.

9. Computer implemented method, according to claim 8, wherein the improvement of a psychological and/or physical state in a subject by providing music therapy comprises reducing the ESAS-r score, reducing symptom distress, reducing the total symptom distress ESAS-r subscale, reducing physical symptoms, reducing the physical score ESAS-r subscale, reducing emotional symptoms, reducing the emotional score ESAS-r subscale, reducing pain, reducing tiredness, reducing drowsiness, reducing nausea, reducing depression, reducing anxiety, ameliorate sleep disturbances, improve well-being and/or reducing boredom.

10. Computer implemented method, according to any of the claims 8 or 9, **characterized in that**:
i) If the system receives in step a) that the subject is suffering from sadness, discouragement and/or loneliness, the system provides a series of five pieces of music with increasing bpm values, wherein the first piece of music is **characterized by** having less than 60 bpm, the second piece of music is **characterized by** having 60-80 bpm, the third piece of music is **characterized by** having 80-100 bpm, the fourth piece of music is **characterized by** having 100-120 bpm, and the fifth piece of music is **characterized by** having more than 120 bpm;
ii) If the system receives in step a) that the subject is suffering from nervousness, worry, anger and/or fear, the system provides a series of five pieces of music with decreasing bpm values, wherein the first piece of music is **characterized by** having more than 120 bpm, the second piece of music is **characterized by** having 100-120 bpm, the third piece of music is **characterized by** having 80-100 bpm, the fourth piece of music is **characterized by** having 60-80 bpm, and the fifth piece of music is **characterized by** having less 60 bpm; or
iii) If the system receives in step a) that the subject is suffering from boredom, weariness and/or apathy, the system provides a series of five pieces of music with increasing bpm values, wherein the first piece of music is **characterized by** having less than 90 bpm, the second piece of music is **characterized by** having 90-110 bpm, the third piece of music is **characterized by** having 110-130 bpm, the fourth piece of music is **characterized by** having 130-150 bpm, and the fifth piece of music is **characterized by** having more than 150 bpm.

11. Computer implemented method, according to any of the claims 8 or 10, further **characterized in that** the last piece is in a major key.

12. Computer implemented method, according to any of the claims 8 to 11, further **characterized in that**:
i) If the system receives in step a) that the subject is suffering from sadness, discouragement and/or loneliness the system provides a series of five pieces of music, wherein the first two pieces of music have a minor key and the last two pieces are in a major key;
ii) If the system receives in step a) that the subject is suffering from nervousness, worry, anger and/or fear the system provides a series of five pieces of music, wherein the last two pieces are in a major key; or
iii) If the system receives in step a) that the subject is suffering from boredom, weariness and/or apathy the system provides a series of five pieces of music, wherein the last piece is in a major key.

13. Computer implemented method, according to any of the claims 8 to 12, wherein the subject is **characterized in that** they have been treated with an anticancer treatment, preferably autologous HSCT, allogeneic HSCT and/or intensive induction chemotherapy for the treatment of AML.

14. Computer implemented method, according to any of the claims 8 to 13, wherein the subject is a patient suffering from AML, a patient that has received an allogeneic stem cell transplant, a patient who has received an autologous stem cell transplant as an inpatient, or a patient who has received an autologous stem cell transplant as an outpatient.

15. A computer implemented program comprising instructions which, when executed by a processing unit, configures the processing unit to execute the computer implemented method according to any one of claims 8 to 14.
